# EUROPEAN PATENT SPECIFICATION

(11) **EP 2 326 377 B1**
(45) Date of publication and mention of the grant of the patent: **29.02.2012**
(21) Application number: 09769700.7
(22) Date of filing: 11.06.2009
(51) Int. Cl.: A61M 16/04, A61M 29/00

(54) **EASY GRIP TAPERED TRACHEOSTOMY DILATOR**
LEICHT ZU GREIFENDER VERJÜNGTER TRACHEOSTOMIE-DILATATOR
DILATATEUR TRONCONIQUE DE TRACHÉOSTOMIE AISÉ À TENIR

(30) Priority: 27.06.2008 US 147817
(43) Date of publication of application: 01.06.2011
(73) Proprietor: KIMBERLY-CLARK WORLDWIDE, INC., Neenah, WI 54956 (US)
(72) Inventor: CUEVAS, Brian, J., Cumming, Georgia 30028 (US); SLEVA, Michael, Atlanta, Georgia 30308 (US); CESA, Joe, Cumming, Georgia 30040 (US); GREENHALGH, Marjory, Decatur, Georgia 30030 (US)
(74) Representative: Davies, Christopher Robert
(86) International application number: PCT/IB2009/052501
(87) International publication number: WO 2009/156890

(56) References cited:
- EP-A1- 1 281 414
- WO-A1-2006/087512
- WO-A1-2008/009943

## Description

Ventilators or respirators are used for mechanical ventilation of the lungs of a patient in a medical setting. The ventilator unit is connected to a hose set; the ventilation tubing or tubing circuit, delivering the ventilation gas to the patient. At the patient end, the ventilation tubing is typically connected to a tracheal ventilation catheter or tube, granting direct and secure access to the lower airways of a patient. Tracheal catheters are equipped with an inflated sealing balloon element, or "cuff", creating a seal between the tracheal wall and tracheal ventilation tube shaft, permitting positive pressure ventilation of the lungs.

One type of tracheal catheter, an endotracheal tube (ET tube), inserted through the mouth, is generally used for a number of days before a decision is made to switch a patient to a tracheostomy tube, inserted directly into the trachea through a stoma in the tracheal wall. Endotracheal tubes have been linked in some studies to an increased rate of ventilator acquired pneumonia (VAP) and so tracheostomy operations are becoming increasingly common and are being performed earlier in the patient's hospital stay in order to reduce the occurrence of VAP.

A tracheostomy procedure involves making a small horizontal incision in the skin of the neck to grant access to the trachea. Because of the uniquely flexible and elastic nature of the trachea, it has been found that healing is much faster if only a small hole is made in the tracheal wall and the hole dilated, rather than cutting the tracheal wall. After the skin incision, a hemostat or other implement may be used to separate the subcutaneous tissues to gain access to the trachea, and digital palpation is used to locate the tracheal rings. A bronchoscope is usually inserted into the ET tube and the tube withdrawn from the trachea until the light of the bronchoscope transdermally illuminates the site of the incision. A sheathed needle is used to puncture the tracheal wall, usually between the second and third tracheal rings. The needle is removed with the sheath remaining, a flexible guide wire (also called a J-wire) is inserted in the place of the needle and the sheath is removed. The bronchoscope is used for viewing the procedure from within the trachea in order to avoid damage to the tracheal wall. A small (e.g. 14 French) introducer dilator is introduced over the guide wire to perform an initial dilation of the tracheal wall, and then removed. A smaller (e.g. 8 French) guiding catheter is then introduced over the guide wire. (Note, French is a measure of circumference based on the theory that non-round tubes of the same circumference will fit into the same incision. One French is approximately 0.33 mm or 0.013 inch).

After the guiding catheter is introduced, a first dilator such as the Cook Medical Inc. Blue Rhino® dilator (see also US patent 6,637,435), is placed over the guide wire and the guiding catheter and first dilator are advanced into the trachea through the tracheal wall as a unit to perform the dilation. Cook Medical recommends a slight over-dilation of the tracheal wall in order to make the placement of the tracheostomy tube easier. After dilation, the first dilator is removed and the tracheostomy tube (with cannula removed) is introduced over the guide catheter using a second, loading dilator that fits just inside the trachostomy tube and protrudes about 2 cm beyond the distal end of the tracheostomy tube. The guide catheter, second dilator and tracheostomy tube are advanced into the trachea through the tracheal wall as a unit. Once the tracheostomy tube is at the proper depth, the second dilator, guide catheter and guide wire are removed through the tracheostomy tube, the inner cannula inserted into the tracheostomy tube and the tube connected to the ventilator. Other dilators are known in WO2006/087512 and EP 1281414 which disclose unitary dilators for use with a tracheostomy tube.

As can be understood from the above description, the current state of the art for tracheostomy involves numerous steps and the insertion and removal of a number of components before the successful completion of the procedure. For most of this time, the patient is disconnected from the ventilator and is therefore, not breathing. In addition, the large number of parts used in current tracheostomy kits increases the likelihood that an item may be accidentally rendered unsterile and be unable to be used. In such cases, the patient must be re-intubated with an ET tube. Even if the procedure proceeds uneventfully, however, the amount of time the patient is not breathing is significant; on the order of 7 minutes or more.

This is clearly a significant event, especially for a patient who is, most likely, not in optimal physical condition.

There remains a need for a device that can more quickly and safely allow for the successful placement of a tracheostomy tube.

### SUMMARY OF THE INVENTION

There is provided a device for performing a tracheostomy. The described tracheostomy dilator has a body and a tip which are detachably attached. The tip has a cannula sized to accommodate a guiding catheter. The tip has a proximal inner portion which is within the body while the tip is attached to the body. After the trachea has been dilated, the body may be detached from the tip and removed and a tracheostomy tube may be inserted over the inner portion of the tip. The tip then may serve to guide a tracheostomy tube into the trachea. After the tracheostomy tube is installed, the tip and other components may be withdrawn through the tracheostomy tube and the tracheostomy tube placed in service.

### BRIEF DESCRIPTION OF THE DRAWINGS

Figure 1 is a drawing of the prior art Blue Rhino® dilator.
Figure 2 is a drawing of the easy grip tapered dilator.
Figure 3 is a drawing of the body or handle portion of the easy grip tapered dilator.
Figure 4 is a drawing of an alternate embodiment of the body or handle portion of the easy grip tapered dilator.
Figure 5 is a drawing of the tip and inner portion of the easy grip tapered dilator.
Figure 6 is a drawing of an alternate embodiment of the tip and inner portion of the easy grip tapered dilator.
Figure 7 is a drawing of the position of a guiding catheter being introduced over a J-wire in the trachea after initial dilation.
Figure 8 is a drawing of the tip of the device being slipped over the guiding catheter through which runs the J-wire.
Figure 9 is a drawing of the device, guiding catheter and J-wire being moved into the trachea through the tracheal wall
Figure 10 is a drawing of the device having been inserted into the trachea through the tracheal wall to the point where the "stop" mark or insertion depth gauge, meets the incision.
Figure 11 is a drawing of the dilator body being removed as indicated by the arrow, leaving the tip, guiding catheter and J-wire.
Figure 12 is a drawing of the dilator tip, guiding catheter and J-wire in place in the trachea after removal of the dilator body.
Figure 13 is a drawing of the tracheostomy tube 26 which has been passed over the inner portion of the dilator tip until it reached the proximal end of the tip where the tube mates with the proximal end of the tip.
Figure 14 is a drawing of the position of the tube and tip as they are passed into the trachea as a unit.
Figure 15 is a drawing of the tip, guiding catheter and J-wire being withdrawn through the tracheostomy tube with the tube remaining in place in the trachea.
Figure 16 is a drawing of the trach tube in its final position in the trachea, with the trach cuff inflated.
Figure 17 is a drawing of the device being immersed in water to activate the lubricious coating, and also showing the alignment line and the surface formation or "grip dimples" that allow for a better hand-hold on the device.
Figure 18 is a drawing of the dilator handle showing the proximal end where the tip locking means is located.

### DETAILED DESCRIPTION OF THE INVENTION

Tracheostomy is a lifesaving procedure to allow a patient to be ventilated directly through the trachea. Tracheostomy is also believed by many to prevent or retard the onset of ventilator acquired pneumonia (VAP). This lifesaving procedure, unfortunately, is relatively time consuming and current technology requires a large number of steps and pieces of equipment that must remain sterile and functioning properly in order to arrive at a successful conclusion.

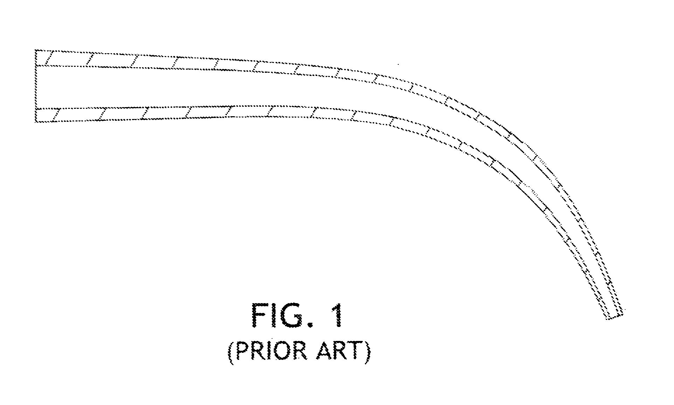

Dilators are instruments or substances for enlarging a canal, cavity, blood vessel or opening, according to the American Heritage Stedman's Medical dictionary 2001. Figure 1 is a drawing of the prior art dilator from Cook Medical Inc. known as the Blue Rhino® dilator (see also US patent 6,637,435). This patent describes a one piece dilator having a generally linear shaft and a short distal tip portion with a curved tapered portion in between.

The tracheostomy procedure may be greatly improved using the device described in the Summary above; the novel easy grip tapered dilator (the device). The device replaces a number of pieces used in the current state of the art procedure described in the introduction. The device replaces both the first and second dilators and so provides fewer steps in the procedure, saving time and reducing risk to the patient. The device also has a number of other novel features to help ensure the consistency and ease of the procedure for the physician. The device is used after the placement of the guiding catheter and J-wire in the trachea.

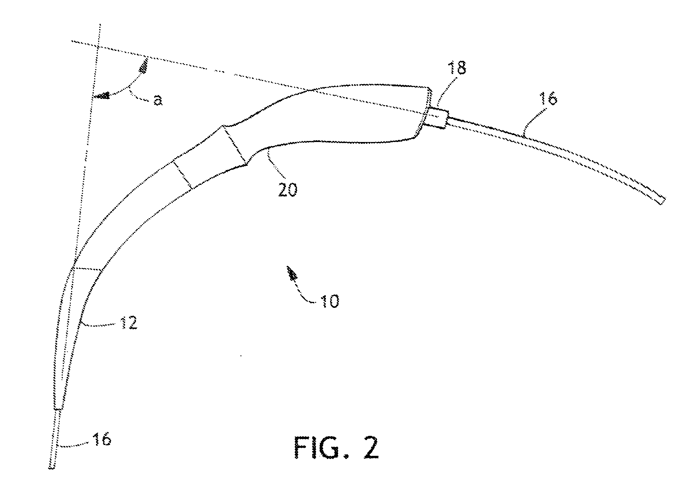

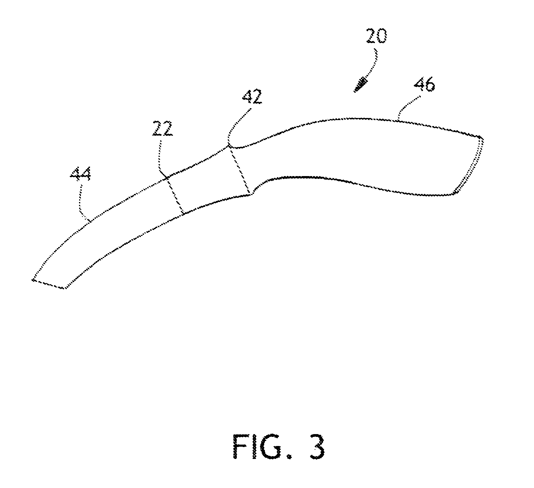

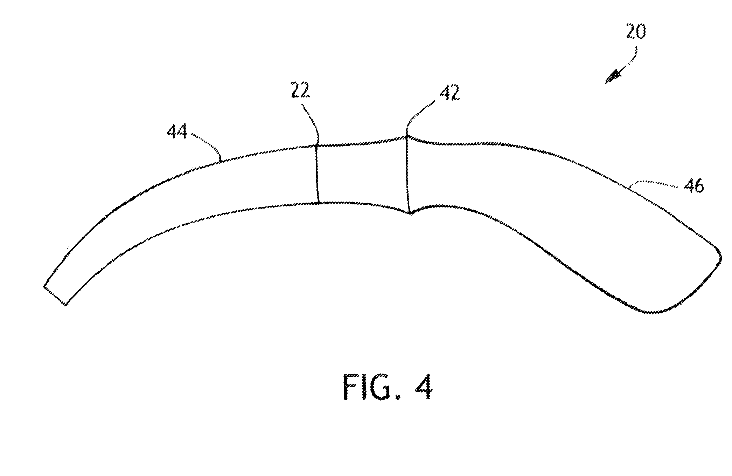

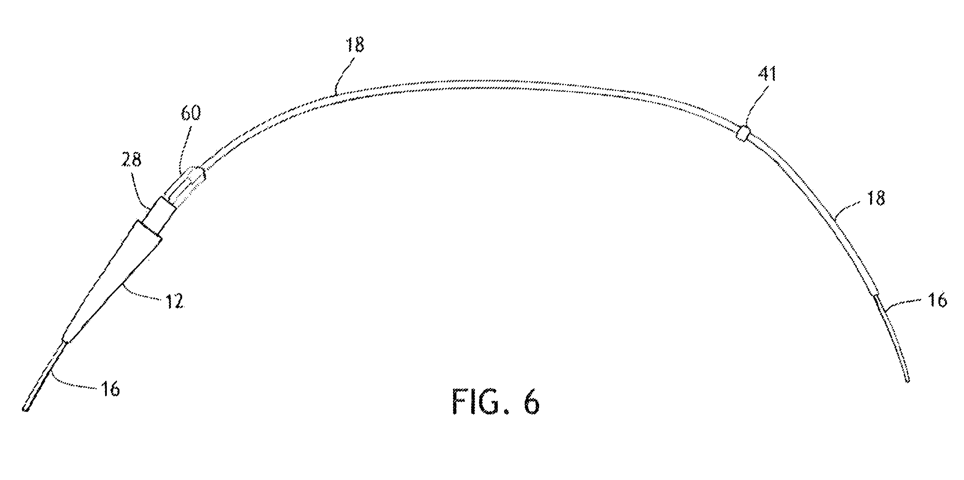

Turning to the Figures, one embodiment of the device 10 has a body 20 and a distal tip 12 (Figure 2) with an inner portion 18. The device 10 has at least two parts or pieces wherein the tip 12 is detachably attached to the body 20. The body 20 is shown in Figure 3 and has a marking line 22 or alternatively a ridge where the diameter is approximately 42 French which serves as a depth marking or insertion stopping point for the dilation procedure. An alternative embodiment of the body 10 to be used with the tip 12 embodiment of Figure 6 is shown in Figure 4. The body 20 has a distal portion 44 and a handle portion 46. The body is sized such that the inner portion 18 of the tip12 can pass through it.

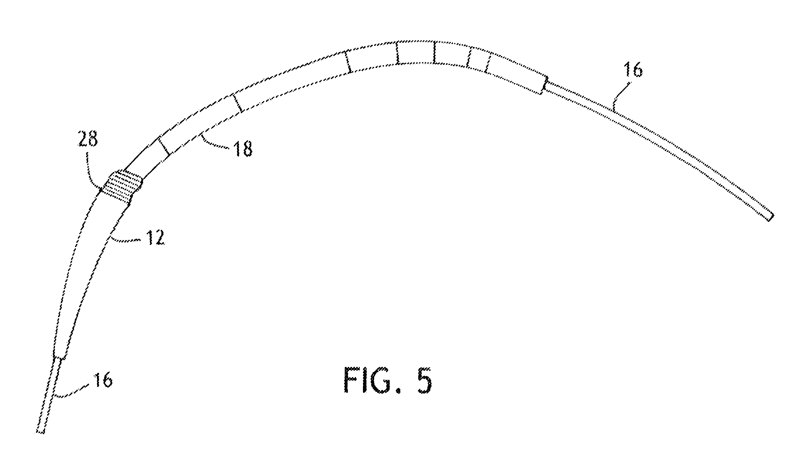

The distal tip 12 meets the body 20 at the proximal end 28 of the tip 12 (Figures 5 and 6). The tip 12 has an proximal inner portion 18 that is surrounded by and passes through the dilator body 20 when the device 10 is comprised of the tip 12 and body 20 connected together. The tip 12 has a cannula sized to accommodate a guiding catheter 14 (not shown) over the J-wire 16 so that the J-wire 16 may pass within the inner portion 18, into the tip 12 and exit the distal end of the tip 12 as shown in Figure 5.

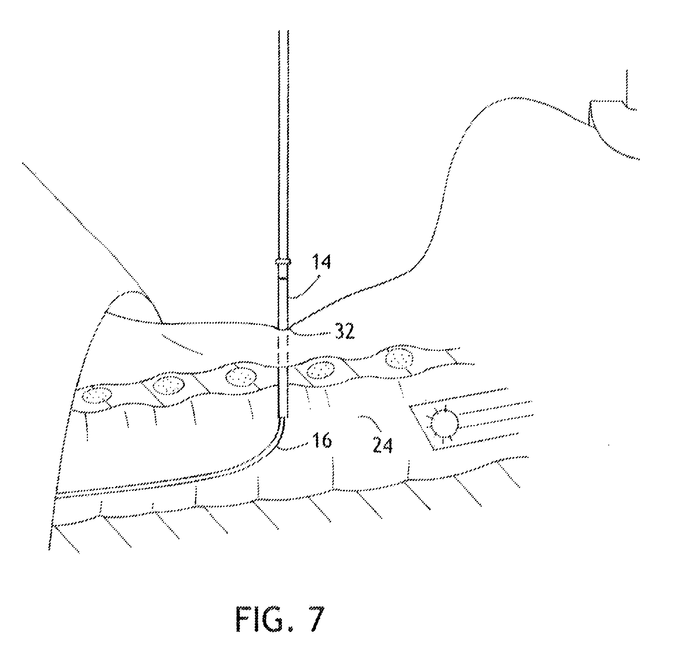

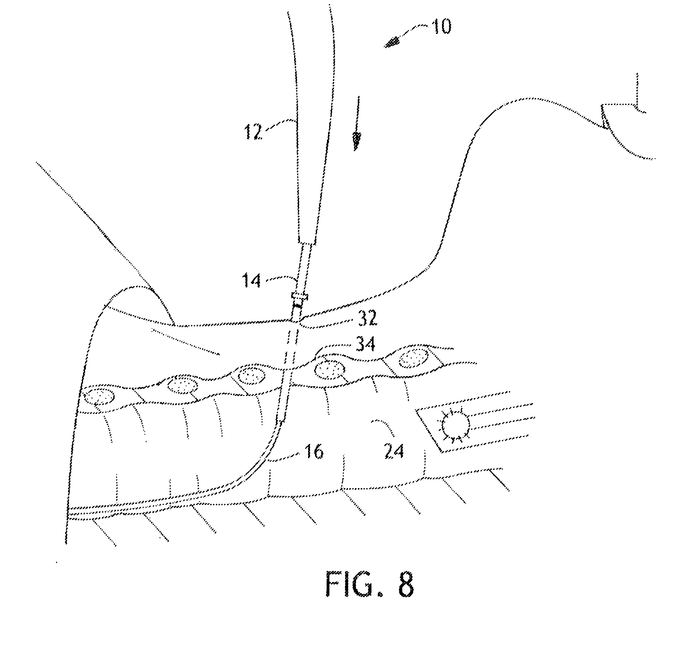

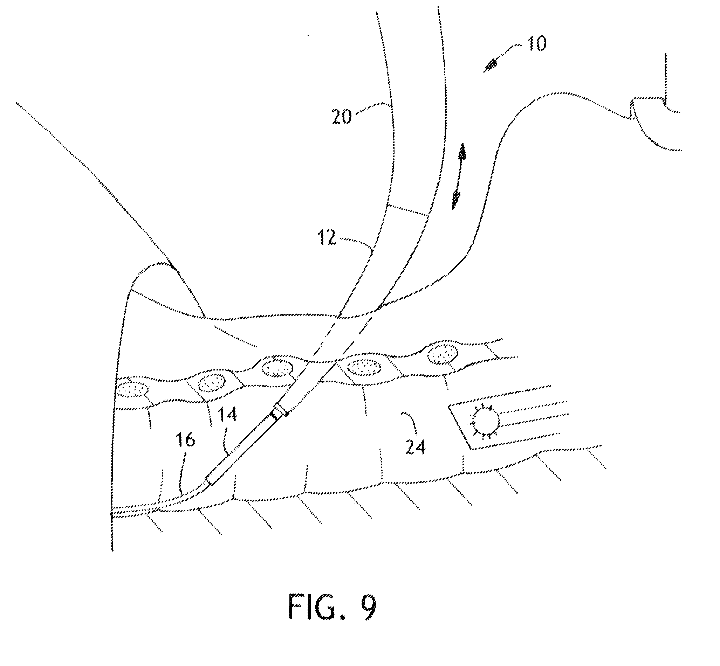

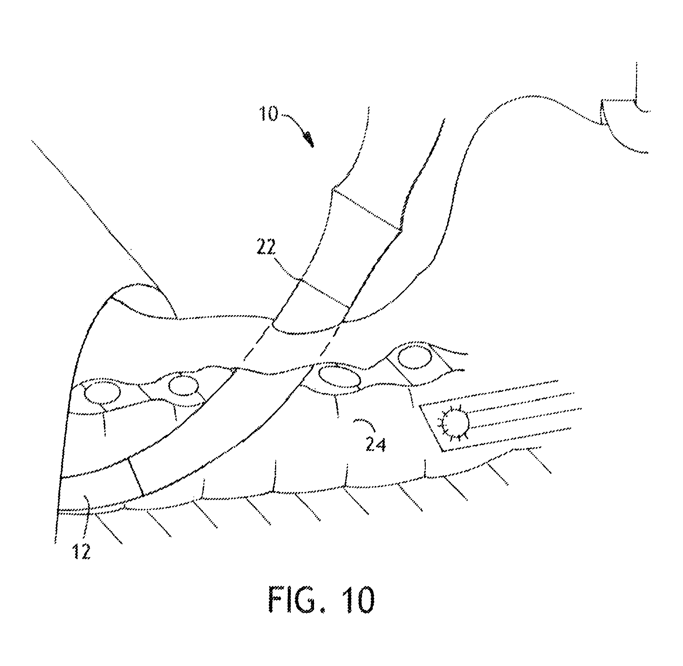

As described above, once the J-wire 16 is inserted into the trachea 24 through the incision 32 and tracheal wall 34, a guiding catheter 14 is introduced over the J-wire 16 (Figure 7). In the tracheostomy procedure using the device 10, the tip 12 of the device 10 is slipped over the guiding catheter 14 through which runs the J-wire 16 (Figure 8). It is also possible to produce the tip 12 of the device 10 such that the tip 12 incorporates the guiding catheter, thus removing the need for a separate guiding catheter (Figure 6). The device 10, guiding catheter 14 and J-wire 16 are then moved into the trachea 24 through the tracheal wall 34 until the marking line 22 of the device 10, which serves as a "stop" mark or depth gauge, meets the incision 32 (Figures 9 and 10 sequentially). The actual procedure of dilation of the tracheal wall usually involves the repeated incremental insertion and removal of the device 10. This procedure may be made easier for the medical provider and less traumatic for the patient by the application of a lubricious coating to the device 10. The coating can reduce friction and drag on the J-wire 16 and also reduce trauma to the area of the incision 32 and the tracheal wall 34. This coating is described in more detail below.

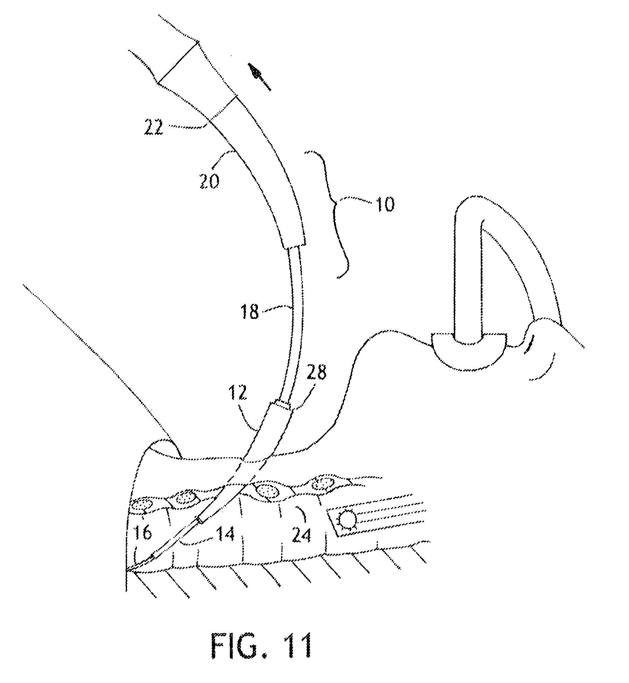

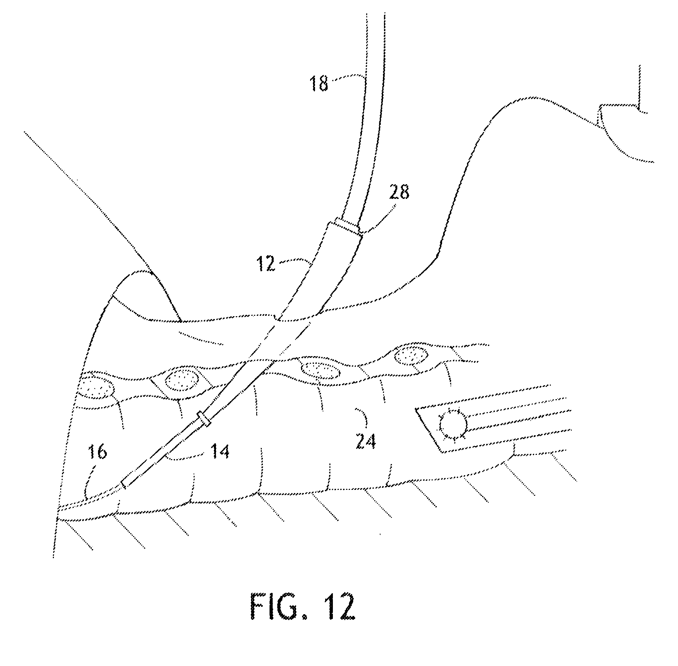

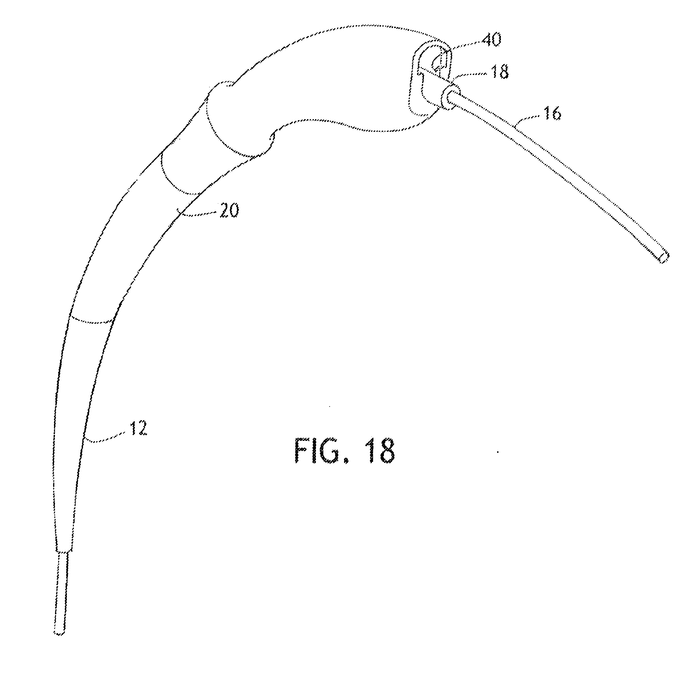

Once the trachea 24 is satisfactorily dilated, the device 10 may be partially removed from the trachea 24, leaving the tip 12 partially, e.g., about half way, into the trachea 24. Note that this view is essentially the same as Figure 9 but occurs after the trachea 24 has been dilated. The dilator body 20 may then be removed as indicated by the arrow in Figure 11, leaving the tip 12, guiding catheter 14 and J-wire 16 in place (Figure 12). The inner portion 18 of the tip12 is also visible in Figure 12. The dilator body 20 may be removed in some embodiments by applying a force perpendicular to the centerline of the dilator body 20 on the guiding catheter proximal locking means (Figure 18). With the guiding catheter 14 no longer held in tension against the proximal locking slot 40 of the dilator body 20 the dilator body 20 can be removed. With the lock released the body 20 is removed by sliding it axially over the proximal end of the guiding catheter 14.

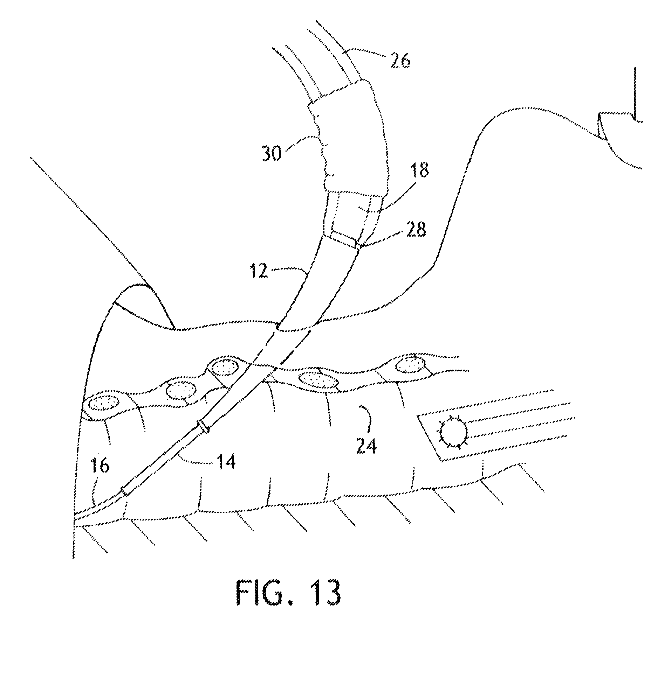

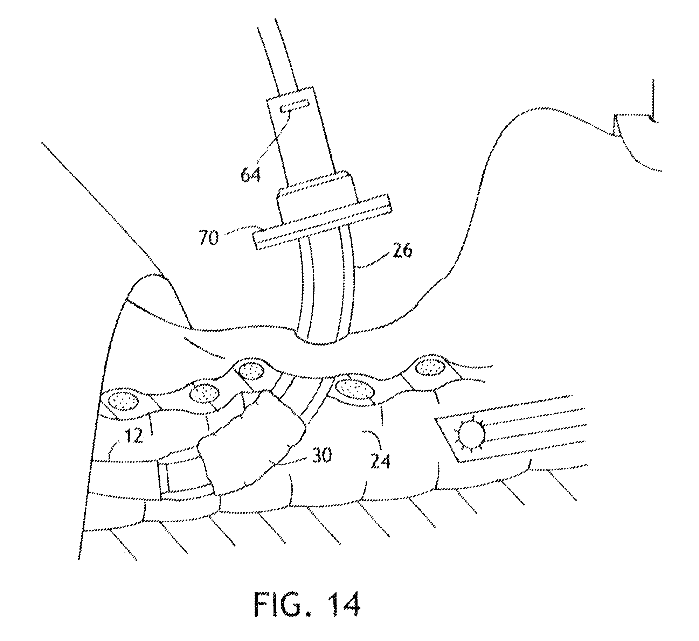

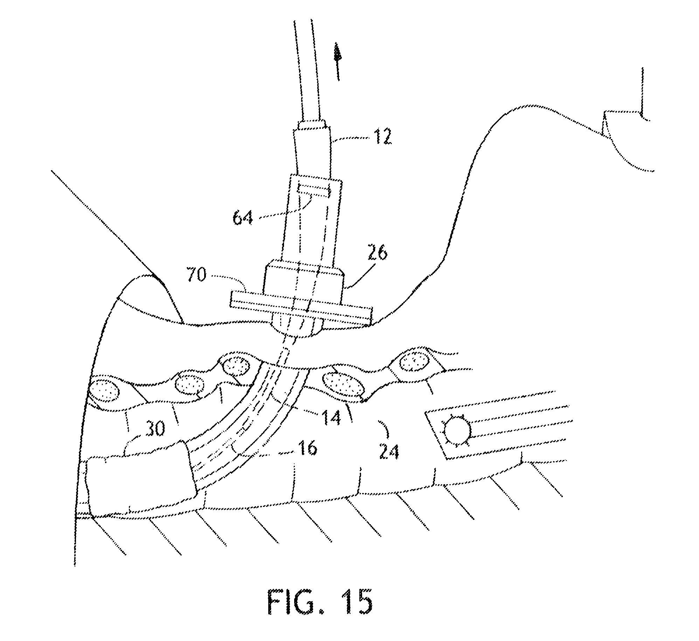

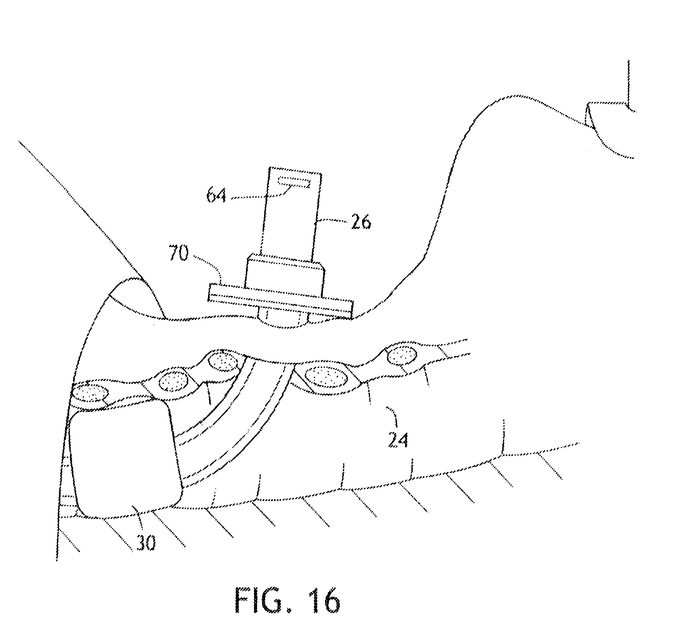

After removal of the body 20, a tracheostomy tube 26 is passed axially over the inner portion 18 of the tip 12 until it reaches the proximal end 28 of the tip 12 where the tube 26 mates with the proximal end 28 of the tip 12 (Figure 13). The tip 12 and tube 26 are then passed into the trachea 24 as a unit (Figure 14). Once the tube 26 is in place in the trachea 24, the tip 12, guiding catheter 14 and J-wire 16 may be withdrawn through the tracheostomy tube 26 with the tube 26 remaining in place in the trachea 24 (Figure 15). A loading catheter like that shown and described in patentee's sister case "Dilator Loading Catheter" filed on the same day as this case may be used to install the trach tube 26 and withdraw the dilator tip 12 if desired. Clearly the tip 12 must be sized so that its largest diameter is slightly less than that of the tracheostomy tube 26 that it is intended to pass through. Lastly, the tube cuff 30 is inflated and the tube 26 is connected to a ventilator (not shown) and placed in service (Figure 16).

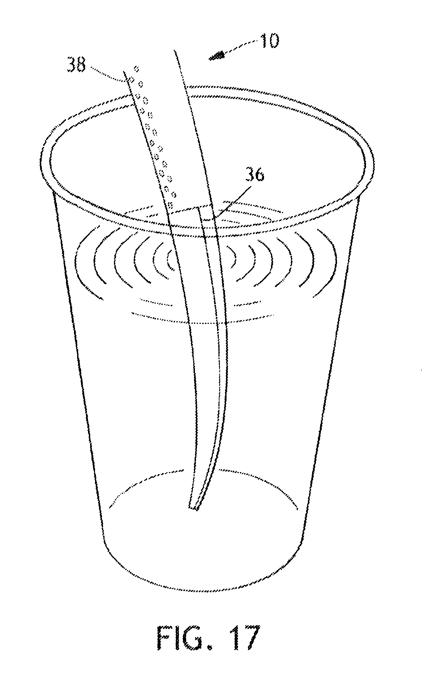

In addition to the above features, the device may have a number of other features to aid the physician in placement of the dilator, some of which are illustrated in Figure 17. One optional feature is a guiding line 36 running length-wise on the uppermost surface of the dilator that allows one to align the J-wire, which also has a line, so that it is facing in the proper direction with the J-loop facing downward. A second optional feature is the use of surface formations 38 to enhance the grip. Such surface formations may be notches, chevrons, "dimples" or other shapes on the dilator body placed in the area where the dilator body would be gripped during a tracheostomy. While the surface formations may be raised from the surface of the dilator body, it is desirable that the surface formations be recessed into the body so as to reduce trauma to tissue. Another optional feature is a ridge 42 located proximal to the marking line 22 as shown in Figures 3 and 4. The dilator body 20 continues to enlarge between the marking line 22 and the ridge 42 so that the tracheal wall may be "over dilated" as preferred by some medical providers. The proximal side of the ridge 42 also serves as a convenient holding point for the user's thumb and fingers during the procedure so that the body 20 may be held like a pencil.

Another optional trauma reducing feature is a lubricious coating that may be added to the tip and dilator body up to the stop ridge on the exterior and/or interior. The coating may be activated by exposure to water (Figure 17) before the device 10 is slipped over the guiding catheter 14. The coating may be for example, a poly(N-vinyl) lactam such as those available from Hydromer Inc., 35 Industrial Parkway, Branchburg, NJ and as described in US patents 5,156,601, 5,258,421, 5,420,197 and 6,054,504. The dilator may be dipped in water just before the J-wire is inserted and may be coated on the inside and/or outside. An inside coating allows the J-wire to slip through the interior of the dilator quite easily and the exterior coating avoids trauma to the skin or trachea.

Still another optional feature is a locking means, discussed briefly above, to hold the inner portion 18 and the rest of the tip 12 in place within the body 20. As seen in Figure 18, the inner portion 18 can fit into a slot 40 in the proximal end of the body 20. There may be matching notches (not shown) placed vertically on either side of the outer surface of the inner portion 18 at the points where the inner portion 18 comes in contact with a ridge or ridges on the inner walls of the proximal end of the body 20 so that the inner portion 18 is locked in place at the point at which the tip 12 is in the proper position. Alternatively, the locking means may include a projection 41 or flange-like structure as shown in Figure 6. The projection 41 is larger than the slot 40. When the tip 12 is fully in place within the body 20 the projection 41 is located proximally beyond the body 20 and keeps the tip 12 and inner portion 18 from distal movement when the inner portion 18 is slid into the slot 40. I.e., the projection 41 acts like a flange to prohibit distal movement of the tip 12 once the inner portion 18 is slipped in to the slot 40. Other means for locking the tip 12 into position within the body 20 include rotary locking means, detents and clamps, for example. The locking means may be located on the proximal end of the tip 12 and the corresponding distal end of the body 20.

Lastly, the marking line 22 at 42 French on the dilator body may instead be a an additional ridge or other marking and alternate or additional markings may be placed on the dilator body at, for example, 32, 38 or still larger French diameters.

The exact size of the dilator device may be varied since the device may be used with adults of varying sizes as well with pediatric patients. A device of a size that functions well with adults may be greatly oversized for use on infants. There are, however, some recommended criteria that should be met. The device, for example, should have a total length of less than 30 cm and weigh less than 35 gms. The dilator tip12 may be between about 25 and 80 mm in length, particularly about 35 mm long, tapering from 3 to 6 mm at the distal end to about 5 to 16 mm, particularly 4 mm at the distal end to 8 mm. The tip inner portion 18 may be between 15 and 30 cm, particularly about 24 cm, in length. The body 20 should be between 12 and 25 cm, more particularly between 15 and 20 cm.

The device should be made from a pliable, flexible material so that it is firm enough to enter the trachea and dilate the tracheal wall, yet not so rigid and firm that it will not flex or "give" when it meets an obstruction. The flexibility of the parts of the device may vary, furthermore, with the distal tip 12 being the most flexible, the proximal end of the body being the least flexible and the flexibility of the body varying between the two. Polymers that may be suitable for use in making the device include polyolefins, polycaprolactones, polyurethanes and others. Polyurethane has been found to be particularly useful in producing the device. The device must be biocompatible, free of di(2-ethylhexyl) phthalate (DEHP) and preferably free of animal derived products.

In contrast to the prior art dilator, the dilator body described herein should be substantially curved (Figure 2) so that the proximal end will have less likelihood of contacting the chin of the patient during the procedure. By "substantially curved" it is meant that while small sections of the device may be or appear straight, the overall shape of the device is clearly curved. As can be seen in Figure 2, for example, the inner portion 18 exits the proximal end of the body 20 at an angle "a" from the tip 12 which, in this instance, is 84 degrees. The degree of curvature may vary, however, and still be considered within the scope of the invention. This angle "a" may be between 110 and 60 degrees, more particularly between 100 and 70 degrees, still more particularly between 90 and 80 degrees.

As will be appreciated by those skilled in the art, changes and variations to the invention are considered to be within the ability of those skilled in the art. Such changes and variations are intended by the inventors to be within the scope of the invention. It is also to be understood that the scope of the present invention is not to be interpreted as limited to the specific embodiments disclosed herein, but only in accordance with the appended claims when read in light of the foregoing disclosure.

## Claims

1. A tracheostomy dilator (10) having a cannula, comprising a body (20) **characterized in that** said dilator further comprises a detachable tip (12), said tip (12) having a proximal inner portion (18) that is within said body (20) while said tip (12) is attached to said body (20).

2. The dilator (10) of claim 1 wherein an angle (a) formed by lines from a proximal end of said body (20) and a distal end of said tip (12) is between 110 and 60 degrees.

3. The dilator (10) of claim 1 wherein an angle (a) formed by lines from a proximal end of said body (20) and a distal end of said tip (12) is between 100 and 70 degrees.

4. The dilator (10) of claim 1 wherein an angle (a) formed by lines from a proximal end of said body (20) and a distal end of said tip (12) is between 90 and 80 degrees, or is between 110 and 60 degrees.

5. The dilator (10) of claim 1 having surface formations on the proximal end (46) of said body (20) to enhance the grip of the user.

6. The dilator (10) of claim 1 further comprising a locking means (40) to hold the tip (12) in place with the body (20).

7. The dilator (10) of claim 6 wherein said locking means (40) is at least one notch on the outer surface of the proximal end of said inner portion of said tip (12), into which fits at least one ridge located on an inner wall on the proximal end (46) of said body (20).

8. The dilator (10) of claim 6 wherein said locking means (40) is a one projection on the outer surface of the proximal end of said inner portion of said tip (12), which is proximal to a slot on the proximal end of said body (20) when said tip (12) is fully in place in said body (20).

9. The dilator (10) of claim 1 having a water activated lubricious coating on a surface.

10. The dilator (10) of claim 1 which changes from relatively very flexible at a distal end to relatively less flexible at a proximal end.

11. The dilator (10) of claim 1 comprising a guiding line (36) running length-wise on an uppermost surface of said dilator (10).

12. The dilator (10) of claim 1 wherein said tip (12) is between 25 and 80 mm in length not including said inner portion (18) which is between 15 and 30 cm and said body (20) is between 12 and 25 cm in length.

13. The dilator (10) of claim 1 wherein said tip (12) incorporates a guiding catheter (14).

14. The dilator (10) of claim 1 wherein said body (20) has a guiding line (36).

15. The tracheostomy dilator (10) of claim 1 wherein said tip (12) is adapted to remain in a trachea (24) when said body (20) is removed during a tracheostomy procedure.

## Patentansprüche

1. Tracheotomie-Dilatator (10) mit einer Kanüle, welcher einen Körper (20) umfasst, **dadurch gekennzeichnet, dass** der Dilatator des Weiteren eine entfernbare Spitze (12) umfasst, wobei die Spitze (12) einen proximalen inneren Anteil (18) aufweist, der sich in dem Körper (20) befindet, während die Spitze (12) an dem Körper (20) befestigt ist.

2. Dilatator (10) gemäß Anspruch 1, wobei ein Winkel (a), welcher durch Linien von einem proximalen Ende des Körpers (20) und einem distalen Ende der Spitze (12) gebildet ist, zwischen 110 und 60 Grad beträgt.

3. Dilatator (10) gemäß Anspruch 1, wobei ein Winkel (a), welcher durch Linien von einem proximalen Ende des Körpers (20) und einem distalen Ende der Spitze (12) gebildet ist, zwischen 110 und 70 Grad beträgt.

4. Dilatator (10) gemäß Anspruch 1, wobei ein Winkel (a), welcher durch Linien von einem proximalen Ende des Körpers (20) und einem distalen Ende der Spitze (12) gebildet ist, zwischen 90 und 80 Grad beträgt oder zwischen 110 und 60 Grad beträgt.

5. Dilatator (10) gemäß Anspruch 1, welcher Oberflächenformationen auf dem proximalen Ende (46) des Körpers (20) aufweist, um den Griff eines Benutzers zu verbessern.

6. Dilatator (10) gemäß Anspruch 1, welcher des Weiteren ein Verschlussmittel (40) umfasst, um die Spitze (12) mit dem Körper (20) in Position zu halten.

7. Dilatator (10) gemäß Anspruch 6, wobei das Verschlussmittel (40) mindestens eine Vertiefung auf der äußeren Oberfläche des proximalen Endes des inneren Teils der Spitze (12) ist, in welche mindestens ein Grat passt, der auf einer inneren Wand des proximalen Endes (46) des Körpers angeordnet ist.

8. Dilatator (10) gemäß Anspruch 6, wobei das Verschlussmittel (40) eine Projektion auf der äußeren Oberfläche des proximalen Endes des inneren Teils der Spitze (12) ist, welche sich proximal eines Schlitzes auf dem proximalen Ende des Körpers (20) befindet, wenn die Spitze (12) komplett in dem Körper (20) in Position ist.

9. Dilatator (10) gemäß Anspruch 1, welcher auf einer Oberfläche eine wasseraktivierbare Gleitbeschichtung aufweist.

10. Dilatator (10) gemäß Anspruch 1, welcher von relativ sehr flexibel an einem distalen Ende zu relativ geringer flexibel an einem proximalen Ende wechselt.

11. Dilatator (10) gemäß Anspruch 1, welcher eine Führungslinie (36) aufweist, die der Länge nach auf einer obersten Oberfläche des Dilatators (10) verläuft.

12. Dilatator (10) gemäß Anspruch 1, wobei die Spitze (12) eine Länge zwischen 25 und 80 mm aufweist, nicht einschließend den inneren Teil (18), welcher eine Länge von zwischen 15 und 30 cm aufweist, und wobei der Körper (20) eine Länge von zwischen 12 und 25 cm aufweist.

13. Dilatator (10) gemäß Anspruch 1, wobei die Spitze (12) einen Führungskatheter (14) beinhaltet.

14. Dilatator (10) gemäß Anspruch 1, wobei der Körper (20) eine Führungslinie (36) aufweist.

15. Tracheotomie-Dilatator (10) gemäß Anspruch 1, wobei die Spitze (12) geeignet ist, in der Trachea (24) zu verbleiben, wenn der Körper (20) während einer Tracheotomie-Prozedur entfernt wird.

## Revendications

1. Dilatateur de trachéostomie (10) ayant une canule, comprenant un corps (20), **caractérisé en ce que** ledit dilatateur comprend en outre un embout détachable (12), ledit embout (12) ayant une partie intérieure proximale (18) qui est à l'intérieur dudit corps (20) alors que ledit embout (12) est attaché audit corps (20).

2. Dilatateur (10) selon la revendication 1, dans lequel un angle (a) formé par des lignes à partir d'une extrémité proximale dudit corps (20) et d'une extrémité distale dudit embout (12) est entre 110 et 60 degrés.

3. Dilatateur (10) selon la revendication 1, dans lequel un angle (a) formé par des lignes à partir d'une extrémité proximale dudit corps (20) et d'une extrémité distale dudit embout (12) est entre 100 et 70 degrés.

4. Dilatateur (10) selon la revendication 1, dans lequel un angle (a) formé par des lignes à partir d'une extrémité proximale dudit corps (20) et d'une extrémité distale dudit embout (12) est entre 90 et 80 degrés, ou est entre 110 et 60 degrés.

5. Dilatateur (10) selon la revendication 1, ayant des formations de surface sur l'extrémité proximale (46) dudit corps (20) pour augmenter la prise de l'utilisateur.

6. Dilatateur (10) selon la revendication 1, comprenant en outre des moyens de blocage (40) pour maintenir l'embout (12) en place avec le corps (20).

7. Dilatateur (10) selon la revendication 6, dans lequel lesdits moyens de blocage (40) sont au moins une encoche sur la surface extérieure de l'extrémité proximale de ladite partie intérieure dudit embout (12), dans laquelle s'adapte au moins une nervure située sur une paroi intérieure de l'extrémité proximale (46) dudit corps (20).

8. Dilatateur (10) selon la revendication 6, dans lequel lesdits moyens de blocage (40) sont une saillie sur la surface extérieure de l'extrémité proximale de ladite partie intérieure dudit embout (12), qui est proximale à une fente sur l'extrémité proximale dudit corps (20) lorsque ledit embout (12) est entièrement en place dans ledit corps (20).

9. Dilatateur (10) selon la revendication 1, ayant un revêtement lubrifiant activé par de l'eau sur une surface.

10. Dilatateur (10) selon la revendication 1, qui passe de relativement très souple à une extrémité distale, à relativement moins souple à une extrémité proximale.

11. Dilatateur (10) selon la revendication 1, comprenant une ligne de guidage (36) s'étendant longitudinalement sur une surface la plus élevée dudit dilatateur (10).

12. Dilatateur (10) selon la revendication 1, dans lequel ledit embout (12) a entre 25 et 80 mm de longueur, non compris ladite partie intérieure (18) qui a entre 15 et 30 cm de longueur, et ledit corps (20) a entre 12 et 25 cm de longueur.

13. Dilatateur (10) selon la revendication 1, dans lequel ledit embout (12) incorpore un cathéter de guidage (14).

14. Dilatateur (10) selon la revendication 1, dans lequel ledit corps (20) a une ligne de guidage (36).

15. Dilatateur de trachéostomie (10) selon la revendication 1, dans lequel ledit embout (12) est adapté pour rester dans une trachée (24) lorsque ledit corps (20) est retiré pendant un protocole de trachéostomie.
